Europäisches Patentamt

European Patent Office

Office européen des brevets

(19)

(11) Numéro de publication: **0 068 055**
**B1**

(12) **FASCICULE DE BREVET EUROPEEN**

(45) Date de publication du fascicule du brevet:
02.04.86

(21) Numéro de dépôt: 81401031.0

(22) Date de dépôt: 26.06.81

(51) Int. Cl.⁴: **A 61 K 35/78,** A 61 K 7/48

(54) Procédé d'obtention d'un extrait stable et désodorisé de sabal serrulatum antiprostatique.

(43) Date de publication de la demande:
05.01.83 Bulletin 83/1

(45) Mention de la délivrance du brevet:
02.04.86 Bulletin 86/14

(84) Etats contractants désignés:
AT BE CH DE GB IT LI LU NL SE

(56) Documents cités:
FR - A - 1 439 044
FR - A - 2 480 754

WIENER KLINISCHE WOCHENSCHRIFT, vol. 91, no. 18,
28 septembre 1979, J. FLAMM et al.: "Urodynamische
Verlaufskontrolle bei Behandlung des Prostataadenoms
mit Phytopräparat und Testosteron", pages 622-627

(73) Titulaire: **P.F. INDUSTRIE, 125, rue de la Faisanderie,
F-75016 Paris (FR)**

(72) Inventeur: **Hatinguais, Philippe, Le Landu Chemin de Bel
air, F-81100 Castres (FR)**
Inventeur: **Belle, René, 73, rue d'Aillot, F-81100 Castres
(FR)**

(74) Mandataire: **Martin, Jean-Jacques et al, Cabinet
REGIMBEAU 26, Avenue Kléber, F-75116 Paris (FR)**

ACTORUM AG

# Description

La présente invention, réalisée au Centre de Recherche PIERRE FABRE, concerne un nouveau procédé permettant d'obtenir un extrait stable et désodorisé de Sabal serrulatum utilisable à titre de principe actif dans des préparations pharmaceutiques.

Le Sabal serrulatum (Michx) est un petit palmier des Etats-Unis, de 1 à 7 m de haut, abondant dans les états du Sud-Est des Etats-Unis, essentiellement entre l'embouchure du Mississipi et la Caroline du Nord. On le rencontre sur des terrains sablonneux arides et incultes.

La partie utilisée pour la réalisation de l'extrait objet de l'invention est le fruit, drupe monosperme ovoïde de 1,5 à 2 cm de long et 1 à 1,5 cm de diamètre, de couleur noir rougeâtre.

La graine est lisse, ovoïde et mate, de 1 cm de long environ et de 0,4 à 0,6 cm de diamètre, à testa dur à embryon latéral dans un albumen homogène.

Les fruits sont récoltés à maturité d'août à janvier suivant les régions.

Jusqu'à présent, les extraits de Sabal serrulatum étaient obtenus par extraction alcoolique. Un extrait fluide de ce type figurant au National Formulary VII p. l37 est préparé classiquement, par macération dans 4 volumes d'éthanol et 1 volume d'eau, suivant le procédé A décrit p. 168. En raison de la richesse en acides gras libres de cette substance végétale, la composition chimique des extraits alcooliques ainsi obtenus évoluait dans le temps par suite des estérifications. En outre, compte tenu de la forte concentration en acides et alcools gras insaturés, ces extraits étaient susceptibles d'oxydation conduisant à une odeur piquante caractéristique.

Le nouveau procédé objet de l'invention pallie les inconvénients énoncés ci-dessus, en permettant d'obtenir un extrait stable, ne modifiant pas l'intégrité des acides et dépourvu d'odeur désagréable.

La présente invention concerne un procédé de préparation d'un extrait stable et désodorisé de fruit du Sabal serrulatum caractérisé en ce que:

a) un broyat de fruit est extrait sous atmosphère inerte par un solvant non polaire inerte vis-à-vis des acides en présence d'un agent antioxydant,

b) on récupère la phase liquide et on élimine le solvant pour obtenir l'extrait huileux contenant les principes actifs.

Parmi les solvants non polaires inertes vis-à-vis des acides il faut citer les hydrocarbures et les hydrocarbures halogénés tels que l'hexane ou l'éther de pétrole, ainsi que les mélanges de ces solvants.

L'extraction est conduite sous gaz inerte par exemple l'azote.

L'antioxydant est choisi parmi le palmitate d'ascorbyle, tocophérols, gallate d'isopropyle, butylhydroxytoluène ou gallate d'isopropyle.

Il est intéressant de noter qu'afin d'améliorer les propriétés du produit, le solvant doit être éliminé totalement, c'est-à-dire que sa concentration doit être inférieure à 1000 ppm de préférence inférieure à 500 ppm.

Le solvant peut être évaporé de préférence sous vide et atmosphère d'azote à une température comprise entre 50 et 120 °C. L'huile obtenue peut être décolorée à l'aide de noir de carbone.

A titre d'exemple non limitatif, il est décrit le mode opératoire industriel suivant:

## EXEMPLE

Les drupes de Sabal serrulatum sont broyées avec un broyeur à disque.

Dans un réacteur de 600 litres, 70 kg de cette poudre végétale, de granulométrie 0,5–1,5 max. sont mis en suspension dans 300 litres d'hexane contenant 5 g de palmitate d'ascorbyle. L'agitation est maintenue une heure en atmosphère d'azote, puis le marc est essoré et lavé par 50 litres d'hexane. Les solutions hexaniques réunies sont préconcentrées dans un appareil à évaporation continue de type LUWA, puis le solvant est fini d'évaporer dans un réacteur sous 25,5 mm de mercure, avec balayage d'azote en continu et chauffage à 60–70 °C.

On obtient ainsi une huile de couleur brunâtre qui est traitée par 2% de charbon de type CECA 2 SA ou 3 SA, à 50 °C environ, sous atmosphère d'azote. Par filtration, on obtient une huile dépigmentée de couleur jaune orangé. L'absence de solvant résiduel est contrôlée par chromatographie en phase vapeur. Le taux minimum est de 300 ppm. Le produit ainsi obtenu possède les caractéristiques suivantes:

– Caractères organoleptiques: liquide limpide à 20 °C, huileux, de couleur jaune orangé
– Densité: voisine de 0,900
– Indice de réfraction: voisin de 1,452 (20 °C)
– Solubilités: soluble dans les solvants organiques et l'alcool, insoluble dans l'eau
– Indice d'iode: compris entre 45 et 55
– Indice de saponification: voisin de 230
– Insaponifiables: 1,8 à 3,5 g/100 g
– Identification des acides gras:

réalisée par CGL: 4% DEGS, sur chromosorb, 3 mètres
T.injecteur-détecteur 250 °C
T.four 100 à 210 °C (4 °C/mn)
acides détectés: $C_6 - C_8 - C_{10} - C_{18}$ (O=)$C_{18}$(2=) et $C_{20}$, et principalement $C_{12} - C_{14} - C_{16}$ et $C_{18}$(1=).

L'analyse du produit objet de l'invention par CPG-SM met en évidence, en plus de βsitostérol antérieurement signalé dans le Sabal serrulatum, du stigmastérol et du campestérol, ainsi que du cycloarténol, de la lupen-3 one, de l'hexacosanol, de l'octacosanol et du triacontanol et des alcools de haut poids moléculaire, compris entre 350 et 600.

Ce produit présente l'avantage d'être stable et dépourvu d'odeur désagréable. Il est utilisable dans des préparations pharmaceutiques utiles au traitement des troubles prostatiques.

Ce produit est également utilisable dans le domaine de la cosmétologie et en dermatologie dans des préparations antiséborrhéiques.

## Revendications

1. Procédé de préparation d'un extrait stable et désodorisé du fruit du Sabal serrulatum caractérisé en ce que:

a) un broyat de fruit est extrait sous atmosphère inerte par un solvant non polaire inerte vis-à-vis des acides en présence d'un agent antioxydant,

b) on récupère la phase liquide et on élimine le solvant pour obtenir l'extrait huileux contenant les principes actifs.

2. Procédé selon la revendication 1, caractérisé en ce que le solvant utilisé est un hydrocarbure, un hydrocarbure halogéné ou un mélange de ces solvants.

3. Procédé selon l'une des revendications 1 et 2, caractérisé en ce que l'antioxydant est choisi parmi le palmitate d'ascorbyle, tocophérols, gallate d'isopropyle, butylhydroxytoluène ou gallate d'isopropyle.

4. Procédé selon l'une des revendications 1 à 3 comprenant un traitement de l'huile avec un noir décolorant.

5. Procédé selon l'une des revendications 1 à 4 dans lequel le produit huileux contenant le principe actif est soumis à un courant d'azote, sous vide, de préférence à une température de 50 à 120°C, de façon à éliminer les impuretés volatiles.

6. Produit obtenu par la mise en oeuvre du procédé selon l'une des revendications 1 à 5.

7. Compositions pharmaceutiques et cosmétologiques contenant le produit selon la revendication 6.

## Claims

1. Process for preparing a stable deodorised extract of Sabal serrulatum fruit, characterised in that:

a) a ground preparation of fruit is extracted under an inert atmosphere by a non-polar solvent which is inert towards acids and in the presence of an antioxidant,

b) the liquid phase is recovered and the solvent removed to obtain the oily extract containing the active principles.

2. Process according to Claim 1, characterised in that the solvent used is a hydrocarbon, a halogenated hydrocarbon or a mixture of these solvents.

3. Process according to one of Claims 1 and 2, characterised in that the antioxidant is chosen from ascorbyl palmitate, tocopherols, isopropyl gallate or butylated hydroxytoluene.

4. Process according to one of Claims 1 to 3, comprising a treatment of the oil with a decolorising charcoal.

5. Process according to one of Claims 1 to 4, in which the oily product containing the active principle is subjected to a stream of nitrogen, under vacuum, preferably at a temperature of 50 to 120°C, so as to remove volatile impurities.

6. Product obtained by implementing the process according to one of Claims 1 to 5.

7. Pharmaceutical and cosmetological compositions containing the product according to Claim 6.

## Patentansprüche

1. Verfahren zur Herstellung eines stabilen und geruchlos gemachten Extraktes der Frucht von Sabal serrulatum, dadurch gekennzeichnet, dass

a) die gemahlene Frucht in Schutzgas mit Hilfe eines nichtpolaren Lösungsmittels, das gegenüber Säuren inert ist, in Gegenwart eines Antioxydans extrahiert wird,

b) man zur Herstellung des öligen Extraktes, das die aktiven Bestandteile enthält, die flüssige Phase gewinnt und das Lösungsmittel eliminiert.

2. Verfahren nach Anspruch 1, dadurch gekennzeichnet, dass das verwendete Lösungsmittel ein Kohlenwasserstoff, ein halogenisierter Kohlenwasserstoff oder eine Mischung dieser Lösungsmittel ist.

3. Verfahren nach einem der Ansprüche 1 und 2, dadurch gekennzeichnet, dass das Antioxydans aus Ascorbylpalmitat, Tocopherol, Butylhydroxytoluol oder Isopropylgallat gewählt wird.

4. Verfahren nach einem der Ansprüche 1 bis 3, umfassend eine Behandlung des Öls mit einer Entfärbungskohle.

5. Verfahren nach einem der Ansprüche 1 bis 4, bei dem das ölige Produkt, das die aktiven Bestandteile enthält, einem Stickstoffstrom, im Vakuum, ausgesetzt wird, vorzugsweise bei einer Temperatur von 50 bis 120°C, sodass die flüchtigen Verunreinigungen eliminiert werden.

6. Produkt hergestellt unter Durchführung des Verfahrens nach einem der Ansprüche 1 bis 5.

7. Pharmazeutische und kosmetische Zusammensetzung, die das Produkt nach Anspruch 6 enthält.